# EUROPEAN PATENT SPECIFICATION

(11) **EP 2 214 736 B1**
(45) Date of publication and mention of the grant of the patent: **05.03.2014**
(21) Application number: 08846165.2
(22) Date of filing: 28.10.2008
(51) Int. Cl.: A61L 27/44, A61L 27/34, A61L 27/54, A61L 27/56, A61F 2/30, A61F 2/34, A61F 2/38

(54) **MEDICAL IMPLANTS AND METHODS FOR DELIVERING BIOLOGICALLY ACTIVE AGENTS**
MEDIZINISCHE IMPLANTATE UND VERFAHREN ZUR ABGABE VON BIOLOGISCHEN WIRKSTOFFEN
IMPLANTS MÉDICAUX ET PROCÉDÉS DE DÉLIVRANCE D'AGENTS BIOLOGIQUEMENT ACTIFS

(30) Priority: 29.10.2007 US 983254 P
(43) Date of publication of application: 11.08.2010
(60) Divisional application: 13163820.7
(73) Proprietor: Zimmer, Inc., Warsaw, IN 46580 (US)
(72) Inventor: FANG, Zhibin, Warsaw IN 46582 (US); SON, Yang, W., Granger IN 46530 (US); VIVANCO, Juan, Madison WI 53726 (US); ZHANG, Kai, Woodbury MN 55125 (US); LEVINE, Danny, L., Mishawaka IN 46544 (US)
(74) Representative: Mays, Julie
(86) International application number: PCT/US2008/081458
(87) International publication number: WO 2009/058780

(56) References cited:
- EP-A1- 1 647 242
- EP-A2- 0 395 187
- US-A- 6 087 553
- US-A1- 2007 179 607

## Description

### BACKGROUND OF THE INVENTION

### 1. Field of the Invention.

The present invention relates to medical implants, such as orthopedic implants of the type used in partial or total joint replacement procedures.

### 2. Description of the Related Art.

Orthopedic implants are used in partial or total joint replacement procedures, such as in hip joint, knee joint, and shoulder joint arthroplasties, for example. Typically, these types of orthopedic implants include a first component associated with a first bone and a second component associated with a second bone, wherein the first and second components articulate with respect to one another. The first and second components may be secured to their respective bones by mechanical interconnection, bone cement, and/or the ingrowth of bone tissue into a porous surface of the implant, referred to as osseointegration.

US 2007/179607 A describes a cartilage resurfacing implant including a body having a bearing surface and a bone interface. Attachment of the implant to the bone may be enhanced by the use of bone growths inducing substances introduced at the bone interface. EP 1 647 242 A1 describes an acetabular cup with a backing layer that may be crated with a bioactive material by sputtering or chemical deposition.

### SUMMARY OF THE INVENTION

The present invention relates to medical implants, as described in claim 1, such as orthopedic implants of the type used in partial or total joint replacement procedures, for example. The implants include a porous substrate, and a bearing portion of a polymeric material which is at least partially molded within the porous substrate. The bearing portion includes a bearing surface that is exposed to an articulating component of another medical implant, and the porous metal substrate contacts the bone for osseointegration of the bone tissue into the porous substrate to anchor the implant. The porous substrate includes biodegradable carrier materials, in the form of one or more layers, that carry biologically active agents such as antibiotics and bone growth factors, for example. The layers of biodegradable carrier materials may be tailored such that, after implantation of the implants, the biologically active agents are released sequentially and/or over time into the surrounding tissue to reduce the chances of infection and/or to promote osseointegration of the implant, for example.

In one form thereof, the present invention provides an implant. The implant includes a porous substrate, a bearing portion of polymeric material, and at least one biologically active agent. The bearing portion is connected to the porous substrate by infiltration of the polymeric material into at least a portion of the porous substrate, and the bearing portion includes a bearing surface. The at least one biologically active agent is incorporated into another portion of the porous substrate.

In another form thereof, the present invention provides a system for incorporating biologically active agents into an implant. The system includes an implant and a mold. The implant includes a porous substrate and a bearing portion of polymeric material connected to the porous substrate by infiltration of the polymeric material into at least a portion of the porous substrate, the bearing portion including a bearing surface. The mold includes a body that conforms to a shape of the porous substrate and at least one channel configured to direct a fluid including at least one biologically active agent into another portion of the porous substrate of the implant.

### BRIEF DESCRIPTION OF THE DRAWINGS

The above-mentioned and other features and advantages of this invention, and the manner of attaining them, will become more apparent and the invention itself will be better understood by reference to the following description of embodiments of the invention taken in conjunction with the accompanying drawings, wherein:
Fig. 1 is a perspective view of an exemplary orthopedic implant, shown as an acetabular cup;
Fig. 2A is a fragmentary sectional view of a portion of the implant of Fig. 1;
Fig. 2B is a schematic representation of Fig. 2A;
Figs. 3A and 3B are depictions of exemplary molding arrangements; and
Figs. 4-7 are further schematic representations of fragmentary sectional views of implants according to alternative embodiments and comparative examples.

Corresponding reference characters indicate corresponding parts throughout the several views. The exemplifications set out herein illustrate embodiments of the invention, and such exemplifications are not to be construed as limiting the scope of the invention any manner.

### DETAILED DESCRIPTION

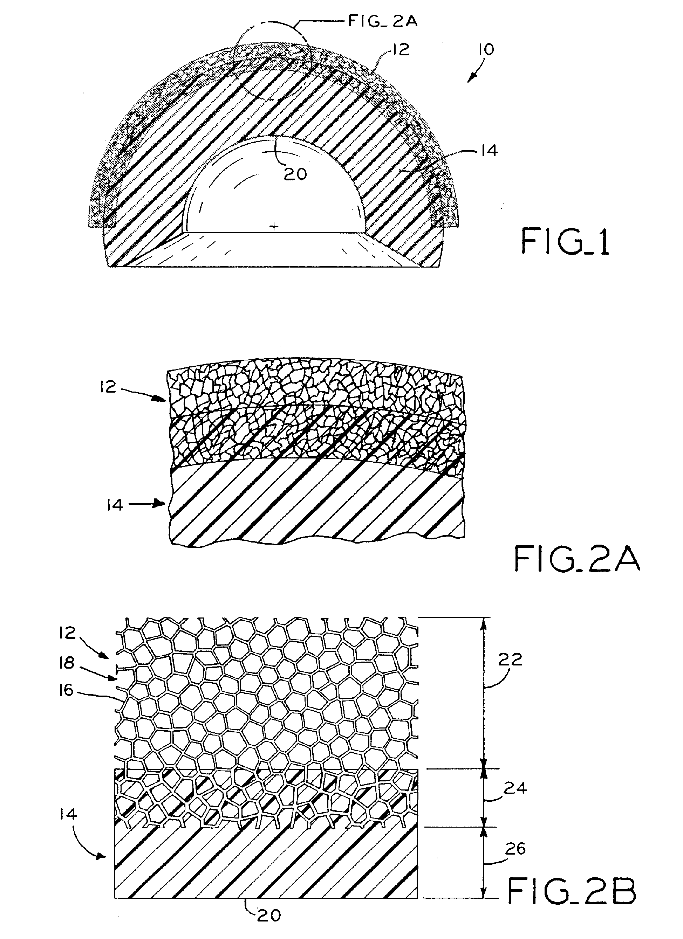

Referring to Fig. 1, an exemplary medical implant is shown in the form of an orthopedic implant and, in particular, an acetabular cup 10 of the type that is implanted within the acetabulum of the pelvis of a patient in a partial or total hip arthroplasty procedure. Acetabular cup 10 generally provides a concave bearing surface that receives the convex articulating head of either the proximal femur itself or of a proximal femoral implant (not shown) that is attached to the femur. Although the present invention is described herein in the form of an orthopedic implant, namely, an acetabular cup, the present invention is generally applicable to any type of medical implant that interfaces with natural tissue, such as bone, when implanted.

Referring to Figs. 1, 2A, and 2B, acetabular cup 10 may be formed as a substantially hemispherical or cup-shaped unitary construct that, as described in detail below, generally includes a porous substrate portion 12 and a bearing portion 14.

Porous substrate portion 12 may be made of a highly porous biomaterial useful as a bone substitute and/or cell and tissue receptive material. An example of such a material is produced using Trabecular Metal™ technology generally available from Zimmer, Inc., of Warsaw, Indiana. Trabecular Metal™ is a trademark of Zimmer Technology, Inc. Such a material may be formed from a reticulated vitreous carbon foam substrate which is infiltrated and coated with a biocompatible metal, such as tantalum, by a chemical vapor deposition ("CVD") process in the manner disclosed in detail in U.S. Patent No. 5,282,861 and in Levine, B.R., et al., "Experimental and Clinical Performance of Porous Tantalum in Orthopedic Surgery", Biomaterials 27 (2006) 4671-4681. In addition to tantalum, other metals such as niobium, or alloys of tantalum and niobium with one another or with other metals may also be used.

Generally, with reference to Fig. 2B, the porous tantalum structure of substrate portion 12 includes a large plurality of ligaments 16 defining open spaces such as voids or channels 18 therebetween, with each ligament 16 generally including a carbon core covered by a thin film of metal such as tantalum, for example. The open spaces between ligaments 16 form a matrix of continuous channels having no dead ends, such that growth of cancellous bone through the porous tantalum structure is uninhibited. The porous tantalum may include up to 75%-85% or more void space therein. Thus, porous tantalum is a lightweight, strong porous structure which is substantially uniform and consistent in composition, and closely resembles the structure of natural cancellous bone, thereby providing a matrix into which cancellous bone may grow to anchor acetabular cup 10 in the surrounding bone of the acetabulum of the pelvis of a patient.

The porous tantalum structure may be made in a variety of densities in order to selectively tailor the structure for particular applications. In particular, as discussed in the above-U.S. Patent No. 5,282,861, the porous tantalum may be fabricated to virtually any desired porosity and pore size, and can thus be matched with the surrounding natural bone in order to provide an improved matrix for bone ingrowth and mineralization.

Bearing portion 14 includes a substantially hemispherical bearing surface 20, and may be formed of a polymeric material such as polyethylene and, in particular, ultra high molecular weight polyethylene (UHMWPE).

Referring to Figs. 2A and 2B, the polymeric material of bearing portion 14 may be molded at least partially within porous substrate 12 to a desired depth to thereby form a unified construct by which the polymeric material of bearing portion 14 is connected to the porous substrate 12 by infiltration of the polymeric material of bearing portion 14 at least partially within the pores or channels 18 of porous substrate 12. In this manner, referring to Fig. 2B, the implant construct generally includes three layers, including a porous layer 22 which will contact and interface with bone tissue when acetabular cup 10 is implanted within a patient, an infiltration layer 24 in which the polymeric material of bearing portion 14 is infiltrated within porous substrate 12, and a bearing layer 26 comprising the polymeric material of bearing portion 14, including bearing surface 20.

As described in detail below, porous layer 22 of the above-described implant construct includes one or more biologically active agents, in the form of one or more layers. After implantation of the implant, the biologically active agent(s) are released or eluted into the surrounding tissue to reduce the chances of infection and/or to promote bony ingrowth, or osseointegration, of bone tissue into porous layer 22 to anchor the implant.

In one embodiment, single or multiple layers of biodegradable carrier materials may be injected into porous layer 22 after bearing portion 14 is molded to porous substrate 12. The biodegradable carrier materials may function as a temporary structural layer to increase the strength of the implant prior to osseointegration, as well as carrier matrix or medium in which the biologically active agent(s) are contained until such time as the implant is implanted. After implantation of the implant, the biodegradable carrier materials will dissolve or resorb into the surrounding tissue, in turn releasing or eluting the biologically active agent(s) into the surrounding tissue.

The biodegradable carrier materials may include biodegradable polymeric materials and/or hydrogels, for example.

Suitable biodegradable polymers that may be used as biodegradable carrier materials include thermoplastic polymers based on poly (ε-caprolactone) (PCL), poly(lactides), or poly(ethylene glycol) (PEG); poly(ortho esters) (POE) and chitosan Poly(DL-lactide), Poly(glycolide), Poly(L-lactide-*co*-glycolide) or Poly(DL-lactide-*co*-glycolide). Natural biopolymers such as chitosan, amphipathic polymers, such as collagen, gelatin and fibrin, and neutral polysaccharides, such as dextran and agarose, may also be used.

Suitable hydrogels that may be used as biodegradable carrier materials include hyaluronic acid, polypropylene fumarate, and Poly(ethylene glycol)-*co*-polylactide, methyl cellulose, and carboxy methyl cellulose. Generally, a hydrogel is a network of polymer chains that are water-soluble but made insoluble through physical and/or chemical crosslinks. These materials are sometimes found as a colloidal gel in which water is the dispersion medium. Hydrogels are generally formed from natural or synthetic polymers. Hydrogels may be classified as "superabsorbent" and may contain over 99% water, by weight. In addition, hydrogels may have the abilty to swell due to water absorption. Hydrogels may also possess a degree of flexibility very similar to natural tissue, due to their significant water content.

Suitable biologically active agents include antibiotics and bone growth factors, for example. Suitable bone growth factors include bone morphogenetic proteins (BMPs) such as BMP-2, -4 and -7, osteoclastogenesis inhibitory factors (OCIF) and geminal bisphosphonates. Suitable antibiotics include Getamicin, Teicoplanin, Aptomycin, Synercid, Linezolid and Tigecycline, for example.

The implant may be designed such that layers that contain antibiotics may be disposed toward the outer regions of the implant that directly interface with, or are positioned proximate, bone tissue, such that the antibiotics are released into surrounding tissues soon after implantation to reduce the possibility of infection and swelling and to promote tissue healing. The biodegradable carrier materials of these layers may be tailored to begin resorbtion, and thereby elution of the biologically active agent(s), within hours or days after implantation, and may require only several hours or a few days, for example, to fully resorb.

Further, the implant may also be designed such that layers that contain bone growth factors may be spaced inwardly from, or beneath, the outer layers of the implant such that, after initial release of antibiotics in the outer layers, bone growth factors are released at a later time to promote full osseointegration of the implant. The biodegradable carrier materials of these layers may be tailored to begin resorbtion, and thereby elution of the biologically active agent(s), after several days or weeks following implantation, and may require several weeks or months, for example, to fully resorb.

In one embodiment, the biodegradable carrier materials and the biologically active agents are mixed and prepared at room temperature or a slightly reduced or elevated temperature, for example, at temperatures that may be as low as 60°, 65°, or 70° F 15.5, 18.3, or 21.1°C, or as high as 75°, 80°, or 85° F 23.8, 26.6, or 29.4°C. The resulting material will typically be a somewhat viscous liquid that may be injected into porous layer 22 of the implant using a suitable injection device, such as a syringe or an injection molding machine, for example. The material then hardens and solidifies to remain stable until implantation.

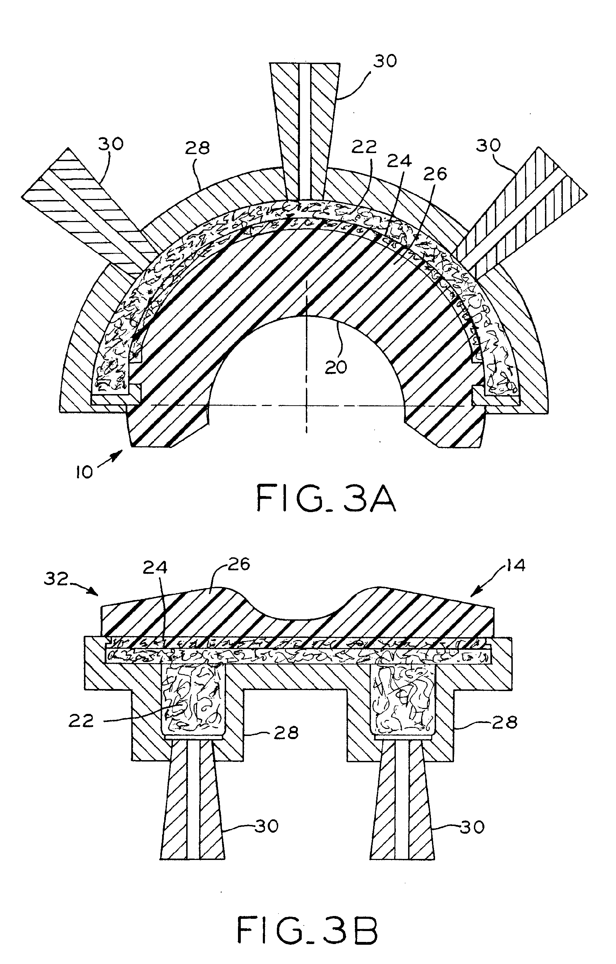

Referring to Figs. 3A and 3B, exemplary depictions of arrangements for direct injection molding of the biodegradable carrier materials into porous layer 22 of implants are shown. In Fig. 3A, porous layer 22 is fitted within a complementary shaped mold body 28, and the biodegradable carrier material is injected through one or more gates or sprues 30 in mold body 28 into porous layer 22. Uniform penetration of the biodegradable carrier material, as well as a desired depth of the biodegradable carrier material, may be achieved by adjusting the pressure, temperature, time, and speed of the injection. A similar molding arrangement is shown in Fig. 3B for another exemplary implant, shown as a tibial implant 32 that includes a porous layer 22 in the form of a tibial base plate and anchor pegs, and a bearing portion 14 against which a distal femoral component (not shown) may articulate.

As discussed below, the implants include multiple layers of biodegradable carrier materials, which may be achieved in one embodiment by using a solvent removal method. In this method, after a single layer of biodegradable carrier material is injected into porous layer 22, a solvent in which the biodegradable material is soluble or partially soluble is applied to the surface of the layer of biodegradable carrier material to remove a portion of the material, thereby reducing or thinning the layer of biodegradable carrier material to a desired depth. A second layer of biodegradable carrier material may then be injected into porous layer 22 above the first layer. If a third layer of biodegradable carrier material is desired, this process may be repeated as described above with respect to the second layer.

In a similar method, a film of polysulfone thermoplastic, for example, can be used to build multiple layers of biodegradable carrier materials in porous layer 22. In this method, a polysulfone film may be impregnated into porous layer 22 from the surface of porous layer 22 to a desired depth from the surface prior to injecting a biodegradable carrier material in between the film and infiltration layer 24, followed by removal of the film using a suitable solvent such as dichloromethane, for example. Optionally, another layer of biodegradable carrier material may then be injected on top of the first layer of biodegradable carrier material in the space previously occupied by the film.

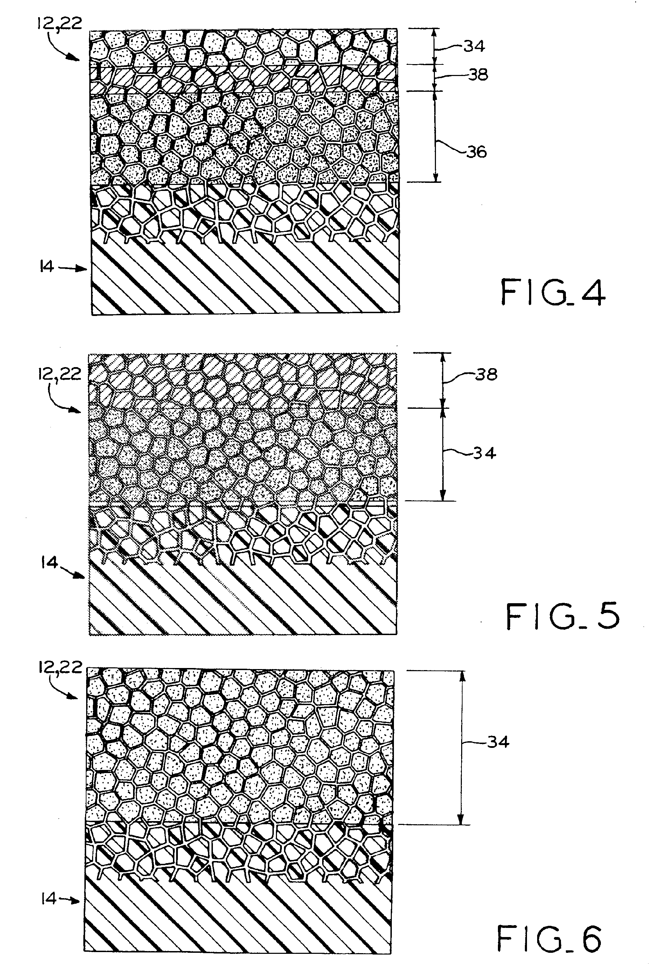

Further exemplary embodiments will now be described with reference to Figs. 4-7. Referring to Fig. 4, in one embodiment, a first layer 34 which, upon implantation of the implant, will be disposed in direct contact with bone, includes a biodegradable carrier material loaded with antibiotics or other pharmaceutical drugs to reduce the possibility of infection and swelling and to promote tissue healing. The resorbtion or elution time of this first layer 28 may be as little as a matter of hours or 1, 2, or 3 days to as long as 1 week, 2 weeks, or 3 weeks, for example.

A second layer 36 is disposed beneath first layer 34 and adjacent the bearing portion 14 of the implant, and may include bone growth factors to promote osseointegration. The resorbtion or elution time of this layer may be as little as 1 week, 2 weeks, or 3 weeks, or as long as 1 month, 2 months, or 3 months, for example.

An optional third layer 38 is disposed between the first and second layers 34 and 36, and may include only a biodegradable carrier material without a biologically active agent. Layer 38 may be tailored to resorb over any of the durations set forth above, and may function as a buffer or barrier layer. In particular, third layer 38 may be tailored to begin resorbtion only after first layer 34 has fully resorbed and eluted its biologically active agent(s), and therefore acts as a buffer layer in the event that full elution of first layer 34 is desired prior to the initiation of the elution of the biologically active agent(s) in second layer 36 to provide a delayed release of the biologically active agent(s) in second layer 36.

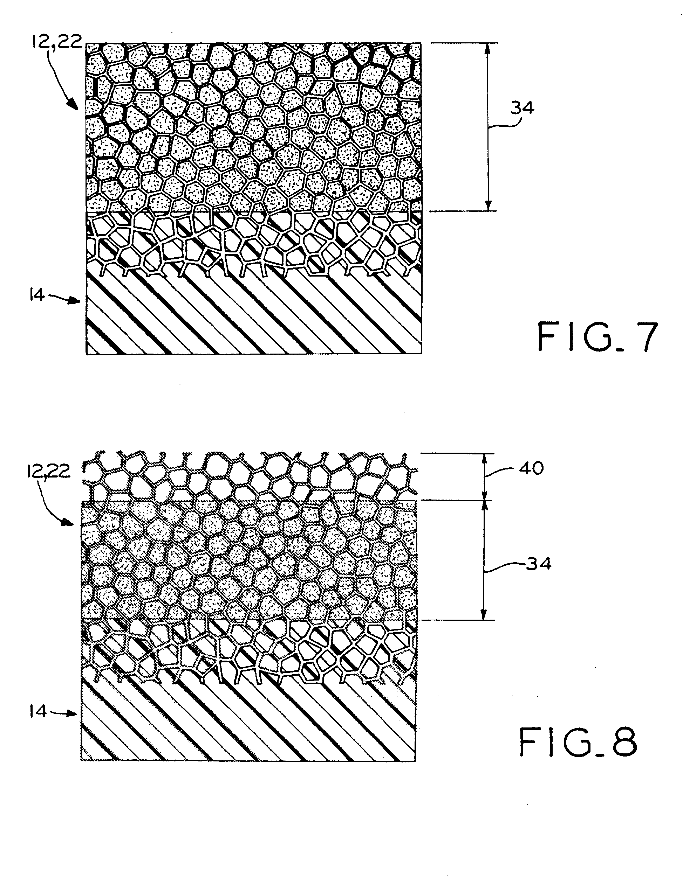

Other configurations are shown in Figs. 5-7. The implant of Fig. 5 includes a barrier layer 38 similar to that of the embodiment of Fig. 4 above, together with a single layer 34 of biodegradable carrier material having one or more biologically active agent(s). Comparative Fig. 6 includes only a single, relatively deep or thick layer 34 of biodegradable carrier material having only biologically active agent(s) in the form of antibiotics, for example. Comparative Fig. 7 includes only a single, relatively deep or thick layer 34 of biodegradable carrier material having only biologically active agent(s) in the form of bone growth factor(s), for example. Comparative Fig. 8 includes an open layer or exposed section 40 of porous portion 12 disposed in contact with the surrounding bone, together with a single, relatively deep or thick layer 34 of biodegradable carrier material having only biologically active agent(s) in the form of bone growth factor(s), for example.

In another embodiment, the initiation of elution, or the speed of elution of the layers of biodegradable carrier material having biologically active agent(s) may be regulated externally of the patient after implantation of the implant using ultrasound, for example, as discussed in co-pending U.S. Provisional Patent Application Serial No. 61/038,852, entitled "Regulation of Medical Device Degradation," filed on March 24, 2008 (Attorney Docket Ref.: ZIM0566). Therefore, the longevity of a porous implant is expected to be increased.

## Claims

1. A medical implant comprising:
a porous substrate;
a bearing portion of polymeric material connected to the porous substrate by infiltration of the polymeric material into at least a portion of the porous substrate, the bearing portion including a bearing surface;
at least one biologically active agent incorporated into another portion of the porous substrate; and
at least one biodegradable carrier material incorporated into the porous substrate, the at least one biodegradable carrier material carrying the at least one biologically active agent;
wherein the at least one biodegradable carrier material is present in the form of a plurality of layers within the porous substrate.

2. The medical implant of claim 1, wherein the at least one biologically active agent is selected from a group consisting of antibiotics and bone growth factors.

3. The medical implant of claim 1, wherein the plurality of layers comprise at least a first layer and a second layer, and wherein the biodegradable carrier material of the first layer carries an antibiotic and the biodegradable carrier material of the second layer carries a bone growth factor, the second layer located between the first layer and the bearing portion of the implant.

4. The medical implant of claim 3, wherein the biodegradable carrier material of the first layer differs from the biodegradable carrier material of the second layer, and wherein the biodegradable carrier material of the first layer has an elution time that is less than that of the biodegradable carrier material of the second layer.

5. The medical implant of claim 1, wherein one of the plurality of layers comprises a barrier layer that delays release of the at least one biologically active agent, whereby the barrier layer lacks a biologically active agent.

6. The medical implant of claim 5, comprising a barrier layer and a single layer of biodegradable carrier material having one or more biologically active agents.

7. A system for incorporating biologically active agents into an implant, the system comprising:
a medical implant comprising:
a porous substrate; and
a bearing portion of polymeric material connected to the porous substrate by infiltration of the polymeric material into at least a portion of the porous substrate, the bearing portion including a bearing surface; and
a mold comprising:
a body that conforms to a shape of the porous substrate; and
at least one channel configured to direct a fluid including at least one biologically active agent into another portion of the porous substrate of the implant.

8. The system of claim 7, wherein the body of the mold is configured to rest directly against the porous substrate of the implant.

9. The system of claim 7, wherein the channel of the mold is configured to receive the fluid under pressure and the body of the mold prevents the pressurized fluid from escaping from the porous substrate of the implant.

10. The system of claim 7, wherein the at least one biologically active agent is selected from a group consisting of antibiotics and bone growth factors.

11. The system of claim 7, wherein the fluid also includes at least one biodegradable carrier material that carries the at least one biologically active agent.

12. The implant system of claim 1 or claim 7, wherein the biodegradable carrier material is a biodegradable polymeric material or hydrogel.

## Patentansprüche

1. Medizinisches Implantat, umfassend:
ein poröses Substrat;
einen Lagerteil aus polymerem Material, der mit dem porösen Substrat durch Infiltration des polymeren Materials in mindestens einen Teil des porösen Substrats verbunden ist, wobei der Lagerteil eine Lageroberfläche einschließt;
mindestens ein biologisch aktives Mittel, das in einen anderen Teil des porösen Substrats inkorporiert ist; und
mindestens ein biologisch abbaubares Trägermaterial, das in das poröse Substrat inkorporiert ist, wobei das mindestens eine biologisch abbaubare Trägermaterial das mindestens eine biologisch abbaubare aktive Mittel trägt;
wobei das mindestens eine biologisch abbaubare Trägermaterial in der Form von einer Vielzahl von Schichten in dem porösen Substrat vorliegt.

2. Medizinisches Implantat nach Anspruch 1, wobei das mindestens eine biologisch aktive Mittel aus einer Gruppe ausgewählt ist, bestehend aus Antibiotika und Knochenwachstumsfaktoren.

3. Medizinisches Implantat nach Anspruch 1, wobei die Vielzahl von Schichten mindestens eine erste Schicht und eine zweite Schicht umfasst, und wobei das biologisch abbaubare Trägermaterial der ersten Schicht ein Antibiotikum trägt und das biologisch abbaubare Trägermaterial der zweiten Schicht einen Knochenwachstumsfaktor trägt, wobei sich die zweite Schicht zwischen der ersten Schicht und dem Lagerteil des Implantats befindet.

4. Medizinisches Implantat nach Anspruch 3, wobei sich das biologisch abbaubare Trägermaterial der ersten Schicht von dem biologisch abbaubaren Trägermaterial der zweiten Schicht unterscheidet, und wobei das biologisch abbaubare Trägermaterial der ersten Schicht eine Elutionszeit aufweist, die kleiner als die des biologisch abbaubaren Trägermaterials der zweiten Schicht ist.

5. Medizinisches Implantat nach Anspruch 1, wobei eine der Vielzahl von Schichten eine Barriereschicht umfasst, welche die Freisetzung des mindestens einen biologisch aktiven Mittels hinauszögert, wobei der Barriereschicht ein biologisch aktives Mittel mangelt.

6. Medizinisches Implantat nach Anspruch 5, umfassend eine Barriereschicht und eine Einzelschicht aus einem biologisch abbaubaren Trägermaterial mit einem oder mehr biologisch aktiven Mitteln.

7. System zum Inkorporieren biologisch aktiver Mittel in ein Implantat, wobei das System Folgendes umfasst:
ein medizinisches Implantat, umfassend:
ein poröses Substrat; und
einen Lagerteil aus polymerem Material, der mit dem porösen Substrat durch Infiltration des polymeren Materials in mindestens einen Teil des porösen Substrats verbunden ist, wobei der Lagerteil eine Lageroberfläche einschließt; und
eine Form, umfassend:
einen Körper, der mit einer Form des porösen Substrats konform ist; und
mindestens einen Kanal, der zum Leiten einer Flüssigkeit, einschließlich mindestens eines biologisch aktiven Mittels, in einen anderen Teil des porösen Substrats des Implantats konfiguriert ist.

8. System nach Anspruch 7, wobei der Körper der Form dergestalt konfiguriert ist, dass er direkt dem porösen Substrat des Implantats anliegt.

9. System nach Anspruch 7, wobei der Kanal der Form dergestalt konfiguriert ist, dass er die mit Druck beaufschlagte Flüssigkeit aufnimmt und der Körper der Form verhindert, dass die mit Druck beaufschlagte Flüssigkeit aus dem porösen Substrat des Implantats entweicht.

10. System nach Anspruch 7, wobei das mindestens eine biologisch aktive Mittel aus einer Gruppe ausgewählt ist, bestehend aus Antibiotika und Knochenwachstumsfaktoren.

11. System nach Anspruch 7, wobei die Flüssigkeit auch mindestens ein biologisch abbaubares Trägermaterial einschließt, welches das mindestens eine biologisch aktive Mittel trägt.

12. Implantatsystem nach Anspruch 1 oder Anspruch 7, wobei das biologisch abbaubare Trägermaterial ein biologisch abbaubares polymeres Material oder Hydrogel ist.

## Revendications

1. Implant médical comprenant :
un substrat poreux ;
une partie d'appui de matériau polymérique raccordée au substrat poreux par infiltration du matériau polymérique dans au moins une partie du substrat poreux, la partie d'appui comprenant une surface d'appui ;
au moins un agent biologiquement actif incorporé dans une autre partie du substrat poreux ; et
au moins un matériau porteur biodégradable incorporé dans le substrat poreux, le au moins un matériau porteur biodégradable portant le au moins un agent biologiquement actif ;
dans lequel le au moins un matériau porteur biodégradable est présent sous la forme d'une pluralité de couches à l'intérieur du substrat poreux.

2. Implant médical selon la revendication 1, dans lequel le au moins un agent biologiquement actif est choisi dans un groupe consistant en des antibiotiques et des facteurs de croissance osseuse.

3. Implant médical selon la revendication 1, dans lequel la pluralité de couches comprend au moins une première couche et une seconde couche, et dans lequel le matériau porteur biodégradable de la première couche porte un antibiotique et le matériau porteur biodégradable de la seconde couche porte un facteur de croissance osseuse, la seconde couche étant située entre la première couche et la partie d'appui de l'implant.

4. Implant médical selon la revendication 3, dans lequel le matériau porteur biodégradable de la première couche est différent du matériau porteur biodégradable de la seconde couche, et dans lequel le matériau porteur biodégradable de la première couche a un temps d'élution qui est inférieur à celui du matériau porteur biodégradable de la seconde couche.

5. Implant médical selon la revendication 1, dans lequel l'une de la pluralité de couches comprend une couche barrière qui retarde la libération du au moins un agent biologiquement actif, moyennant quoi la couche barrière est exempte d'agent biologiquement actif.

6. Implant médical selon la revendication 5, comprenant une couche barrière et une couche unique de matériau porteur biodégradable ayant un ou plusieurs agents biologiquement actifs.

7. Système d'incorporation d'agents biologiquement actifs dans un implant, le système comprenant :
un implant médical comprenant :
un substrat poreux ; et
une partie d'appui de matériau polymérique raccordée au substrat poreux par infiltration du matériau polymérique dans au moins une partie du substrat poreux, la partie d'appui comprenant une surface d'appui ; et
un moule comprenant :
un corps se conformant à une forme du substrat poreux ; et
au moins un canal configuré pour diriger un fluide comprenant au moins un agent biologiquement actif dans une autre partie du substrat poreux de l'implant.

8. Système selon la revendication 7, dans lequel le corps du moule est configuré pour reposer directement contre le substrat poreux de l'implant.

9. Système selon la revendication 7, dans lequel le canal du moule est configuré pour recevoir le fluide sous pression et le corps du moule empêche le fluide sous pression de s'échapper du substrat poreux de l'implant.

10. Système selon la revendication 7, dans lequel le au moins un agent biologiquement actif est choisi dans un groupe consistant en des antibiotiques et des facteurs de croissance osseuse.

11. Système selon la revendication 7, dans lequel le fluide comprend en outre au moins un matériau porteur biodégradable qui porte le au moins un agent biologiquement actif.

12. Système d'implant selon la revendication 1 ou la revendication 7, dans lequel le matériau porteur biodégradable est un matériau polymérique biodégradable ou un hydrogel.
